# EUROPEAN PATENT SPECIFICATION

(11) **EP 2 133 053 B1**
(45) Date of publication and mention of the grant of the patent: **24.08.2016**
(21) Application number: 09162489.0
(22) Date of filing: 11.06.2009
(51) Int. Cl.: A61F 13/49, A61F 13/15

(54) **Disposable undergarment with improved elasticised sections**
Einweg-Unterwäsche mit verbesserten elastischen Abschnitten
Sous-vêtement jetable avec sections élastisées améliorées

(30) Priority: 11.06.2008 EP 08158000
(43) Date of publication of application: 16.12.2009
(73) Proprietor: Ontex BVBA, 9255 Buggenhout (BE)
(72) Inventor: Smet, Steven, 9240 Zele (BE); De Poorter, Annick, 9320 Erembodegem - Aalst (BE)
(74) Representative: Brants, Johan P.E.

(56) References cited:
- EP-A- 0 692 233
- EP-A- 1 027 874
- EP-A- 1 208 827
- DE-A1-102005 030 182
- US-A1- 2005 055 001

## Description

### Field of the invention

The present invention relates to a disposable undergarment and, more particularly, to a disposable absorbent undergarment such as a disposable diaper, incontinence pads, training pants, menstrual flow pants and the like.

### Background of the invention

Disposable absorbent undergarments are well-known in the art. They generally comprise an absorption body and a textile-like outer wrap into which the absorption body is contained. The outer wrap is generally in pants shape, with two openings for each one of the legs and a larger opening acting as a waist opening. Such products can be provided with leakage resistance improving features such as upstanding containment flaps or cuffs as taught in US 4,808,178, US 4,695,278, US 4,795,454, US 5,085,654 and others. The challenge presented to the designers of such disposable absorbent undergarment products is to reconcile two key requirements: providing excellent comfort for the user of such products and creating optimal resistance against the leakage of body fluids from the product.

It is a well known practice to attach elastic members to the foresaid undergarments to improve comfort and leakage resistance and many patterns thereof have been in use. This well known practice of attaching elastic members typically allows for different conditions thereof; for example, the types, the number, the arrangement spaces and the contractile forces of the elastic members. Some elastic member positioning techniques have the objective to provide good fit by creating a chain of elastically stretchable elements around at least the inner part of the legs.

For example, EP 0692 233 by Fujioka teaches to provide an undergarment with third elastic members being stretchable longitudinally of the undergarment and intersecting first and second elastic members extending along front- and rear-halves of leg-hole defining edges, respectively, so that these first, second and third elastic members may cooperate with one another to surround the respective leg-hole defining edges without interruption.

Alternatively, EP 0 446 867 by Igaue teaches to combine the elastic elements at the leg opening with third elastic elements in the upstanding cuff so that after fitting on the body of the wearer, elastically stretchable lines formed by the leg-hole elastic elements in places intersect elastically stretchable lines formed by the third elastic elements.

Document US 2005/055001 A1 discloses a disposable absorbent underpant, which comprises first, second and third pairs of leg elastics secured between an outer cover and an inner liner, and arranged around each of a pair of leg openings. Each of the third pair of leg elastics is separated from one of the first pair of leg elastics by a first gap and from one of the second pair of leg elastics by a second gap.

Document DE 10 2005 030182 A1 discloses a pant-like disposable absorbent article comprising a peripherally continuous waist edge forming a waist opening and leg openings, front piece and back piece with longitudinal side edge sections and a crotch region between the front and back pieces. Waste edge and leg openings are obtained by joining the longitudinal side edge sections of a front and back piece. The article includes an absorbent body with band elements on both sides. Every leg opening has an elastic section. Each band element has a nonelastic band section and when the product is in use elastic section crosses the respective nonelastic band section.

Document EP 1 027 874 A discloses an integral disposable pull-up absorbent article which has elasticated front and back waist portions, an insert member having an absorbent core comprising an elasticated crotch region and at least one elastic member in the longitudinal direction on each side of the crotch region.

Document EP 1 208 827 A discloses a liquid impervious core arranged in a crotch region which includes front and rear end zones extended from a middle zone towards front and rear waist regions. The elastic portions include side portions extending from front and rear ends, middle portions extending on side of a liquid impervious back sheet across front and rear end zones of core.

Such techniques have the disadvantage of not presenting an optimal resistance against the leakage of bodily fluids, especially since the discharge of bodily fluids can be sudden and quite heavy. The techniques as described by Igaue and Fujioka want to ensure a good contact between the product and the body of the wearer, thus working only indirect on preventing bodily liquids from leaking. Typical attempts to improve leakage resistance of such constructions consist in increasing the contractile force of the elastic members in order to obtain an even tighter contact between the product and the body of the wearer. However, so increased contractile force of the elastic members may disturb blood circulation around the legs, create rash and generally deteriorate the comfort of the undergarment to wear. Another important disadvantage of providing a too tight fit along an uninterrupted chain of elastically contractible elements is that it hinders the exchange of air between the inside of the product and the environment, thus creating an unfavourable microclimate inside the product, giving rise to sweaty feelings or even skin irritations.

### Summary of the invention

It is the objective of the present invention to provide an undergarment addressing the above problems, and in particular an undergarment which has an excellent resistance against the leakage of bodily fluids but remains comfortable to the wearer at all times, allows for better aeration of the crotch area and/or is cost-efficient in manufacturing.

In particular, the inventors realised an improved arrangement of elastic elements on disposable undergarments of the above type, whereby the crotch area can be shaped as a cup or pocket. Such cup form of the crotch area can contain even one or more discharges of bodily liquids which are heavy in both flow and volume, allowing them to be gradually absorbed by the absorbing core underneath.

To achieve the objective set forth above, the invention broadly resides in a disposable absorbent undergarment comprising: a liquid-permeable topsheet; a liquid-impermeable backsheet; a liquid-absorbent core between the topsheet and the backsheet; a laminate comprising the topsheet, the backsheet and the core and having a front section, a rear section and a crotch section interposed between the front section and the rear section; first and second leg-hole defining edges in the form of generally circular-arc-shaped notches on transversely opposite sides of the crotch section; stretchable elastic members generally extending along each of the first and second leg-hole defining edges; the elastic members comprising first and second front-half elastic portions extending primarily along first and second front-halves of the first and second leg-hole defining edges, first and second rear-half elastic portions extending primarily along first and second rear-halves of the first and second leg-hole defining edges, and first and second core shaping elastic portions extending longitudinally on transversely opposite sides of the crotch section, wherein said first core shaping elastic portion does not intersect with any one of said first front-half and first rear-half elastic portions, and said second core shaping elastic portion does not intersect with any one of said second front-half and second rear-half elastic portions.

According to the present invention, the first and second core shaping elastics have the function of creating or at least facilitating the formation of a cup in the absorbent body in the crotch area, thus efficiently reducing the risk of leakage of bodily fluids, whereas the first and second front and rear elastics have as a main function providing a good fit around the legs of the wearer and increasing comfort. Without limitation, the inventors suggest that absence of an intersection between the core shaping elastics and at least one or both of the respective front- and rear-elastics means that the contractile force is not divided or distributed over a continuous chain of intersecting elastics encircling the leg openings. Rather, the contractile force exerted by the core shaping elastics can act substantially independently to induce a cup form within the crotch area. The formation of a cup is important in that such a cup has the shape of a valley, a pocket or other type of depression in the absorbing core so that liquids are being drawn to the bottom of that cup by the forces of gravity and see themselves surrounded by higher walls or barriers formed by the areas of the absorbing core closer to the edges. These higher walls or barriers are liquid-impermeable as the absorbing core is typically constructed with a liquid impermeable layer underneath. Gravity thus pulls the liquids into the lowest depression and the vertical walls or barriers keep them there so that the absorbing materials inside the absorbing core have time to take up the liquids and store them. In case no such cup shape would be present, then the liquids could run along the surface of the absorption core which has only a limited liquid absorption speed, towards the edges of the absorption core and could create leakage. In addition, the absence of intersection between the core shaping elastics and at least one or both of the respective front- and rear-elastics provides for a relatively non-elasticised area between said elastics, which more loosely encloses a wearer's body and thus favours good blood circulation in the upper thigh area. It thereby also allows for improved air exchange (aeration) between the inside of the product and the environment.

Hence, it may be contemplated in the present invention that the first and second front-half elastic portions form a front elasticised area, the first and second core shaping elastic portions form a core elasticised area, and the first and second rear-half elastic portions form a rear elasticised area, with a relatively non-elasticised area between said front and core elasticised areas and a relatively non-elasticised area between said rear and core elasticised areas.

The invention provides a disposable absorbent undergarment comprising: a liquid-permeable topsheet; a liquid-impermeable backsheet; a liquid-absorbent core between the topsheet and the backsheet; a laminate comprising the topsheet, the backsheet and the core and having a front section, a rear section and a crotch section interposed between the front section and the rear section; first and second leg-hole defining edges in the form of generally circular-arc-shaped notches on transversely opposite sides of the crotch section; stretchable elastic members generally extending along each of the first and second leg-hole defining edges; the elastic members comprising first and second front-half elastic portions extending primarily along first and second front-halves of the first and second leg-hole defining edges, first and second rear-half elastic portions extending primarily along first and second rear-halves of the first and second leg-hole defining edges, and first and second core shaping elastic portions extending longitudinally on transversely opposite sides of the crotch section, wherein said first core shaping elastic portion does not intersect with any one of said first front-half and first rear-half elastic portions, and wherein said second core shaping elastic portion does not intersect with any one of said second front-half and second rear-half elastic portions, characterised in that said first core-shaping elastic portion extends longitudinally beyond or outwardly relative to the crotch end of the first front-half and/or first rear-half elastic portions, and the second core-shaping elastic portion extends longitudinally beyond or outwardly relative to the crotch end of the second front-half and/or second rear-half elastic portions.

This arrangement provides for comparably longer core shaping elastic portions which allow the crotch area to form a containment pocket and wherein body discharge is effectively guided to said pocket through a longitudinally central section of the absorbent article delimited by the comparably longer crotch elastic portions.

Without intending to limit the present invention, the present invention will in a preferred embodiment create a disposable absorbent undergarment which is even more useful and leakage resistant against loose faecal material and gushes of urine, if the leg-hole elastic members creating a leg-hole elasticised area are attached in a semi-circular, curved or horseshoe-like pattern around the leg opening defining edges.

Research has thus indicated that, as leg-hole elasticised sections and core shaping sections according to the present invention have largely different functions, both will work most efficiently if each of them is allowed to operate substantially independently from the other sections, i.e. substantially without residual tensions or forces coming from the other elasticised areas and disturbing the own elasticised section's force vectors' directions and magnitudes.

Apart from providing a better functionality, the present invention with elasticised sections operating substantially independently from one another also allows for a more purpose-specific raw material use and thus cost saving versus designs already known in the art.

### Brief description of the drawings

**Fig. 1** is a perspective view showing an embodiment of a disposable absorbent undergarment according to the invention;
**Fig. 2** is an exploded perspective view showing the undergarment as longitudinally developed;
**Fig. 3** is a plan view showing a first embodiment of a core shaping elastic arrangement used in the diaper;

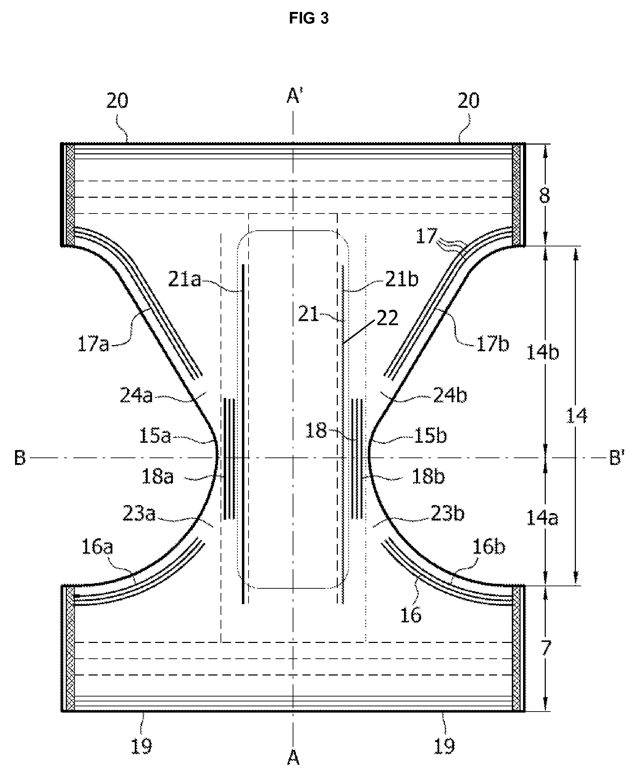

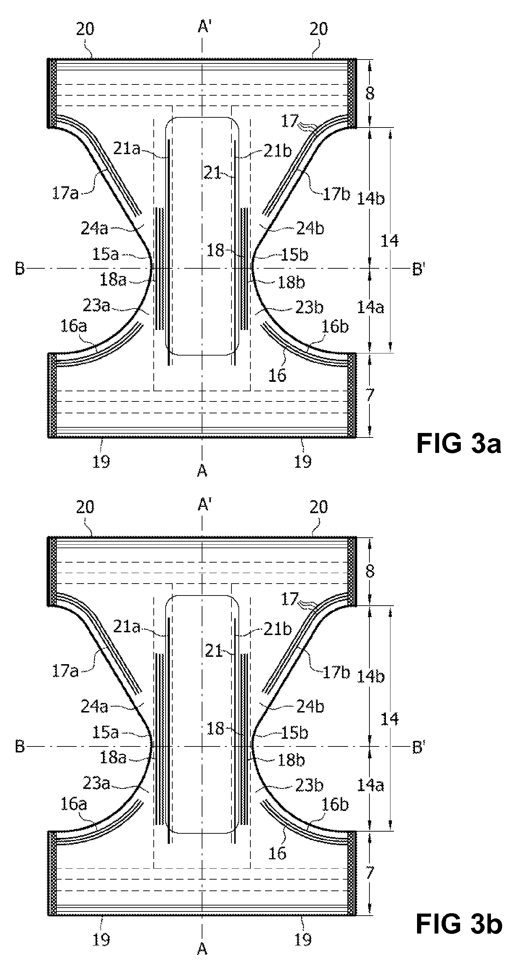

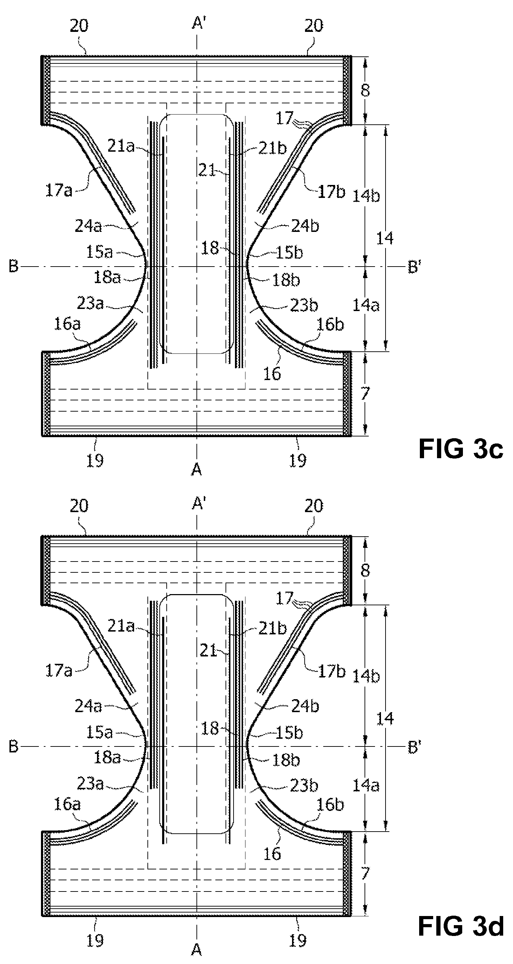

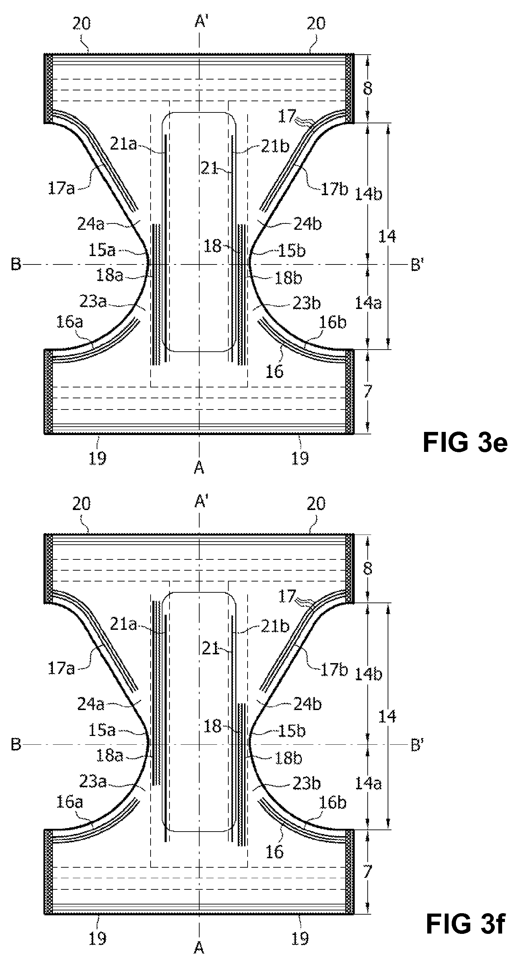

**Fig. 3 a-f** present plan views showing embodiments of a core shaping elastic arrangement comprising comparably longer core shaping elastic portions, used in the diaper of the invention;

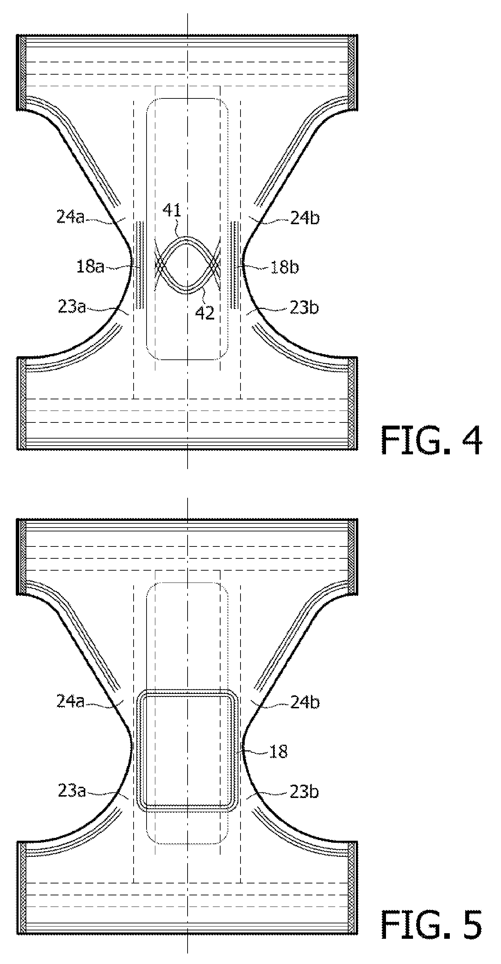

**Fig. 4** is a view similar to Fig. 3, showing a second embodiment of the core shaping elastic arrangement;
**Fig. 5** is a view similar to Fig. 3, showing a third embodiment of the core shaping elastic arrangement;

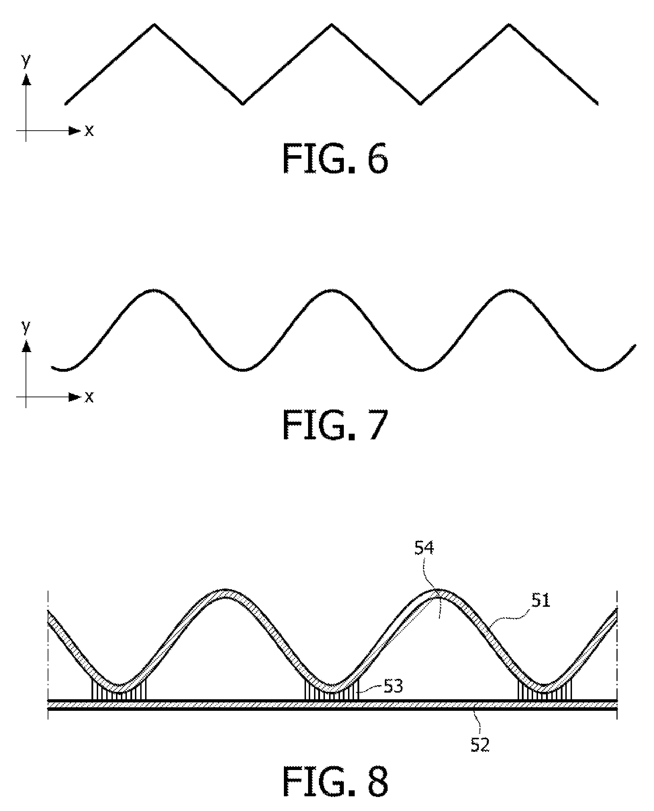

**Fig. 6** is a view of an elastic member pattern for one of the elasticised sections.
**Fig 7** is a view of an alternative embodiment of an elastic member pattern for one of the elasticised sections.
**Fig. 8** is a view of a cross-section of a non-elasticised area allowing improved airflow and cup formation.

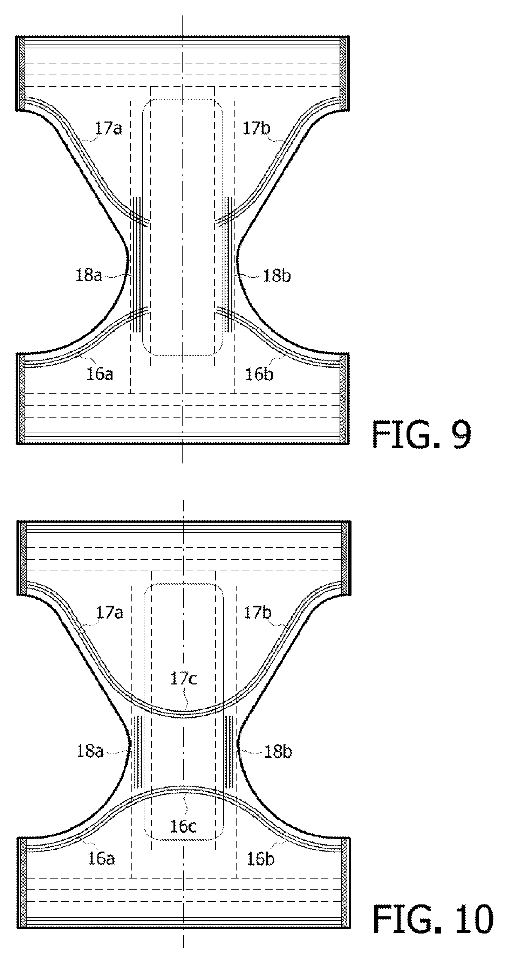

**Fig. 9** is a view similar to Fig. 3, showing a further embodiment of the core shaping elastic arrangement.
**Fig. 10** is a view similar to Fig. 3, showing a further embodiment of the core shaping elastic arrangement.

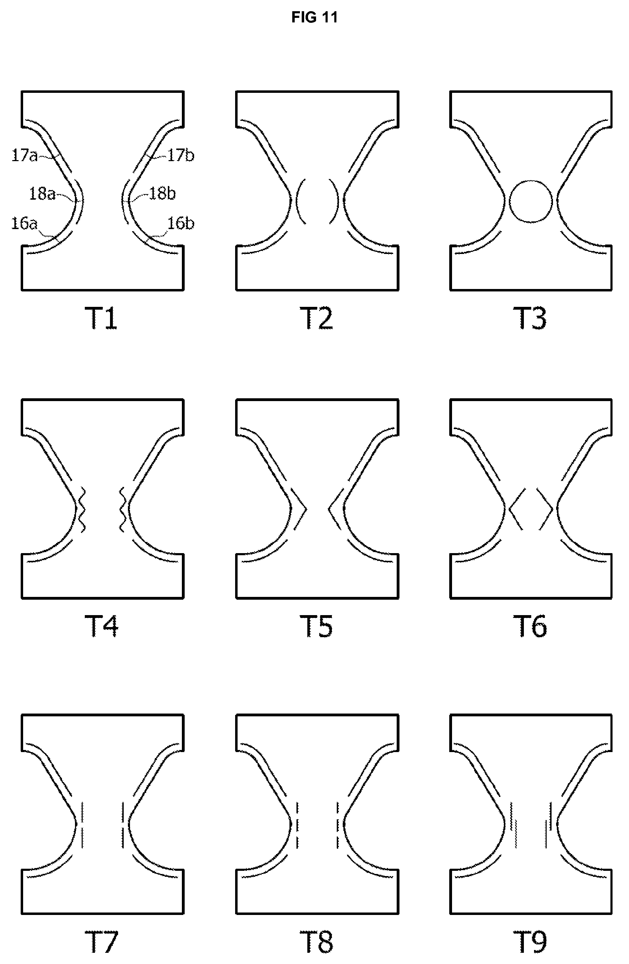

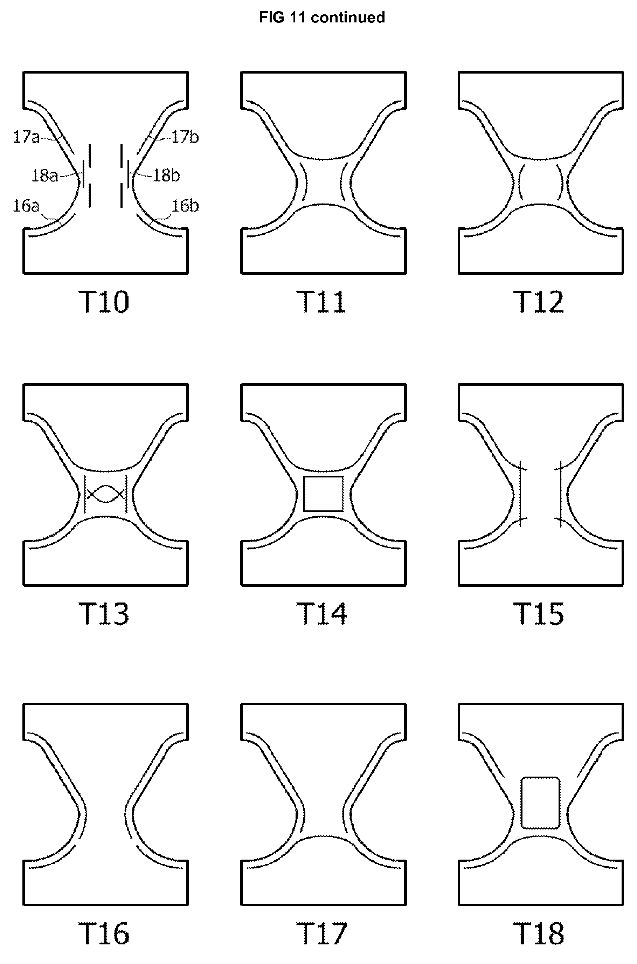

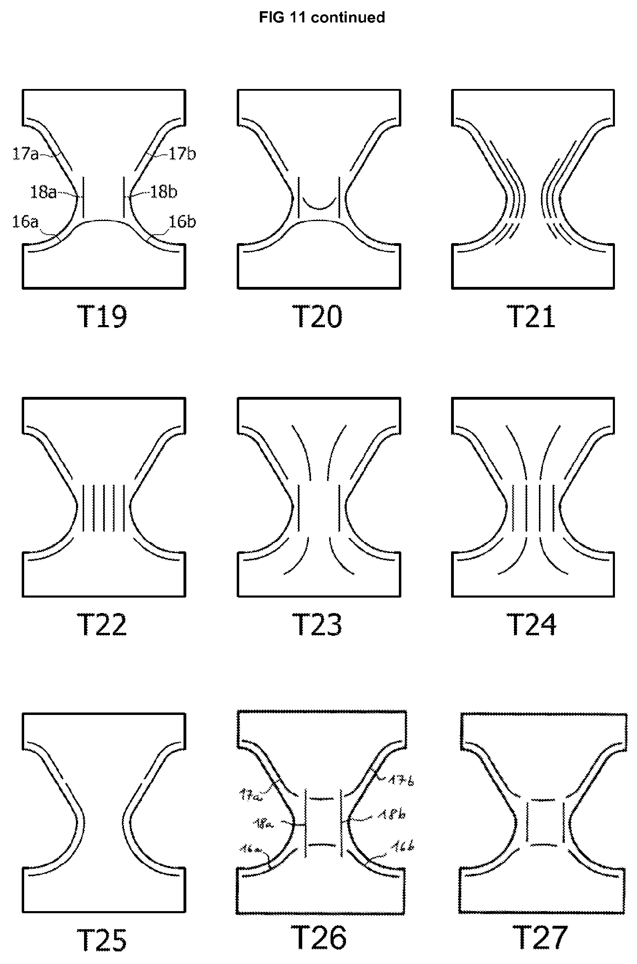

**Fig. 11** is a view similar to Fig. 3, schematically illustrating embodiments of the invention with various arrangements of the elastic members 16, 17 and 18.

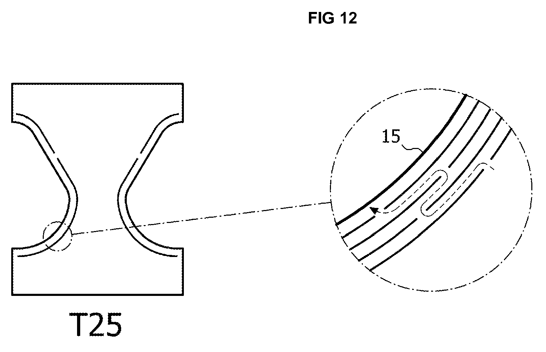

**Fig. 12** illustrates, in connection with the arrangement T25, a further design of the crotch and/or leg elastic members having irregularly or unequally spaced gaps in the elastic members or strands thereof to further hinder the escape of liquids (dashed arrow) from the crotch region through the edges 15.

### Detailed description of the invention

As used herein, the singular forms "a", "an", and "the" include both singular and plural referents unless the context clearly dictates otherwise. By way of example, "an elastic member" refers to one or more than one elastic members.

The terms "comprising", "comprises" and "comprised of" as used herein are synonymous with "including", "includes" or "containing", "contains", and are inclusive or open-ended and do not exclude additional, non-recited members, elements or method steps.

The terms "attach" and "attached" as used herein are synonymous with their counterparts of the terms "join", "fasten", "affix", "secure", "glue", "bind" and "link". Typical examples of methods used to attach two materials to each other are via the use of an adhesive such as a pressure sensitive adhesive or via the use of ultrasonic or other thermal, mechanical or thermo-mechanical bonding techniques.

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. However, it is to be understood that the value to which the modifier "about" refers is itself also specifically disclosed.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released.

The terms "stretching", "stretchable", "stretchability", etc. refer to materials and objects comprised thereof that are extensible when forces are applied to the material, and offer some resistance to extension. In the present specification, where stretching of elastic materials is indicated, this will typically refer to such stretching within the elastic range of the stress-strain function of the said materials, i.e., below the yield value above which such stretching would already contain a component of plastic deformation. Hence, after such stretching (i.e., elastic stretching), the elastic material will resume substantially to its original dimensions.

With regard to the direction in which the elastic members are said to extend on the undergarment, it is not necessary for the elastic members to run parallel to a certain axis X (refer to Fig. 6 and 7 for illustration) for the main forces of the respective elasticised section to "extend primarily" along direction X and thus to result in forces running primarily in a direction parallel to said axis X. Even though the elastic members may also induce force components running along the Y-axis, some of them may compensate each other and/or the residual or overall component along the Y-axis (if any) will be smaller than the resulting force along the X-axis.

By means of example, when an elastic member such as a core shaping elastic member is said to extend longitudinally with respect to the absorbent article, the overall elastic or contractile force or pull along the transverse centreline (illustrated as axis BB' in Fig. 3), if any, will be smaller than the overall elastic force along the longitudinal axis of the article (illustrated as axis AA' in Fig. 3). For example, the overall elastic force along the longitudinal axis may be at least 2-fold, more preferably at least about 5-fold, even more preferably at least about 10-fold, still more preferably at least about 20-fold, yet more preferably at least about 50-fold, and very preferably at least about 100-fold, at least about 200-fold, at least about 500-fold or at least about 1000-fold greater than the overall elastic pull (if any) along the transverse axis.

The following further explains the invention, with particular reference to several exemplary but non-limiting embodiments.

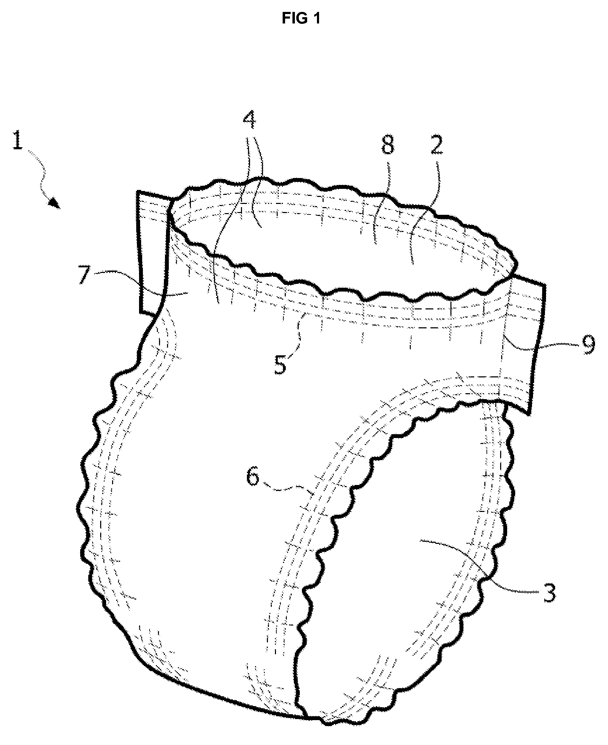

Referring to Fig. 1, an absorbent undergarment 1 is in the form of pants type and has a waist-hole 2 and a pair of leg-holes 3 formed on a textile-like outer wrap 4. In a preferred embodiment, this outer wrap is a laminate of materials providing a soft and tactile appearance. The waist-hole 2 is circumferentially provided with a waist surrounding elastic member 5 and the leg-holes 3 are also circumferentially provided with leg surrounding elastic members 6, respectively. Front and rear bodies defining front and rear sections 7, 8 of the diaper 1 lie one upon another along transversely opposite side edges and are integrally bonded to each other by bonding lines 9.

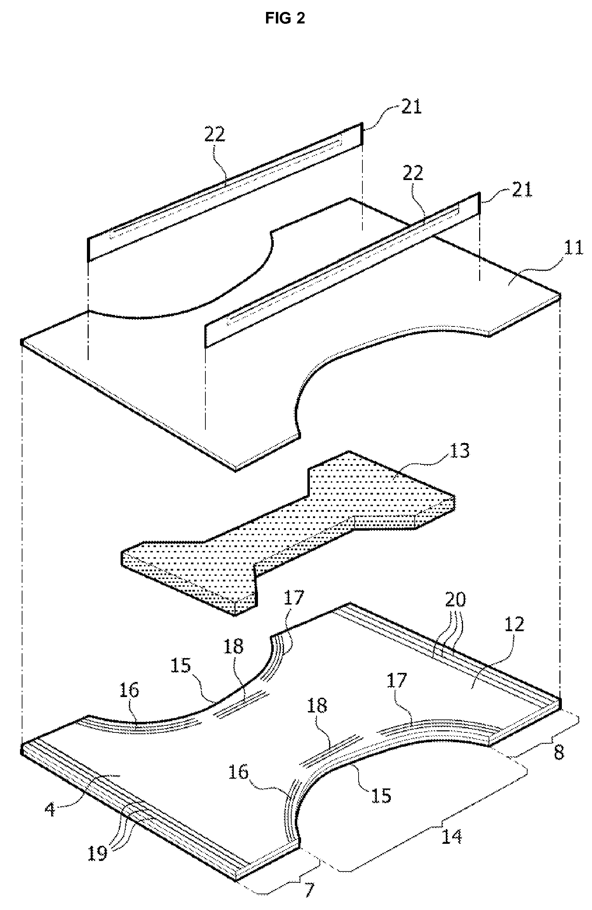

Referring to Fig. 2, the absorbent undergarment 1 comprises, as viewed in the direction of thickness, a laminate outer wrap 4 including a liquid-permeable topsheet 11, a liquid-impermeable backsheet 12 and a liquid-absorbent core 13 disposed between these two sheets 11, 12. Portions of the top- and backsheets 11, 12 extending outward beyond a peripheral edge of the core 13 are water-tightly bonded together and the core 13 is intermittently bonded to at least one of the sheets 11, 12. The diaper 1 is longitudinally composed of the front and rear sections 7, 8 and a crotch section 14 interposed there between. The crotch section 14 is formed along transversely opposite sides with a pair of circular-arc-shaped notches destined to define leg surrounding edges 15, respectively. First (front-half), second (rear-half) and third (core shaping) elastic members 16, 17 and 18 are attached to the inner surface of the backsheet 12 facing the topsheet 11 in association with the respective leg-hole defining edges 15. Of the first, second and third elastic members 16, 17 and 18 each comprising single or plural elastic strings, the first and second elastic members 16, 17 are attached to the backsheet 12 with a tension directed at least partly along a pair of leg-hole defining edges 15 and the third elastic members 18 are attached to the backsheet 12 with a tension at least partly longitudinally of the diaper 1 and allowing for easy cup formation so as to act as core shaping elastic members. Fourth elastic members 19 and 20 are attached to the inner surface of the backsheet 12 facing the topsheet 11 in association with the respective edges defining waist opening 4. As additional protection against the sideways leakage of bodily fluids or loose faecal material, a pair of upstanding cuffs 21 containing fifth elastic members 22 is attached to the topsheet 11.

The absorbent core 13 may be any means which is generally compressible, conformable, non-irritating to the wearer's skin, and capable of absorbing and retaining liquids and certain body exudates. A preferred absorbent core 13 has a garment surface and a body surface and comprises an absorbent layer. The absorbent layer may be manufactured in a wide variety of sizes and shapes (e.g., rectangular, hourglass, I-shaped, T-shaped, etc.). It may be preferred that the absorbent core be narrower in the crotch region. The absorbent layer may be manufactured from a wide variety of liquid absorbent materials commonly used in disposable diapers and other absorbent articles, such as comminuted wood pulp. Examples of other suitable absorbent materials include creped cellulose wadding, absorbent foams, cellulosic acetate fibers, absorbent sponges, super absorbent polymers, absorbent gelling materials, or any equivalent materials or combination of materials. The total absorbent capacity of the absorbent layer should, however, be compatible with the design exudate loading in the intended use of the undergarment 1. Further, the size and absorbent capacity of the absorbent core 13 may be varied to accommodate wearers ranging from infants through adults.

The absorbent core 13 may include a matrix of hydrophilic fibres, such as a web of cellulosic fibres, mixed with particles, fibres or a compound of a high-absorbency material such as that commonly known as super absorbent material. The term "high-absorbency material" refers to materials that are capable of absorbing at least 10 times their own weight in liquid. The absorbent core may comprise a mixture of super absorbent hydrogel-forming particles and wood pulp fluff. The wood pulp fluff may be exchanged with synthetic, polymeric, melt-blown fibres or with a combination of melt-blown fibres and natural fibres. The high-absorbency material may be substantially homogeneously mixed with the hydrophilic fibres or may be non-uniformly mixed. The high-absorbency material may also be arranged in a generally discrete layer within the matrix of hydrophilic fibres. Alternatively, the absorbent core may comprise a laminate of fibrous webs and high-absorbency material or other suitable means of maintaining a high-absorbency material in a localized area. The high-absorbency material may be selected from natural, synthetic and modified natural polymers and materials. The high-absorbency materials may be inorganic materials, such as silica gels, or organic compounds, such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations such as hydrogen bonding, and hydrophobic associations or Van der Waals forces. Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl ethers and alpha-olefins, poly(vinyl pyrolidone), poly(vinyl morpholinone), poly(vinyl alcohol), and mixtures and copolymers thereof. Further polymers suitable for use in the absorbent core include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthum gum, locust bean gum, guar gum, gelatine and the like. Mixtures of natural and wholly or partially synthetic absorbent polymers can also be useful in the present invention. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core.

The absorbent article may further comprise materials which can provide for odour control. On one hand, such materials may be able to mask the often unpleasant odour of body exudates. Various odour-controlling agents are known in the art and can be employed in the present invention. Exemplary materials may include certain zeolitic materials which are known for their odour-controlling properties. Advantageously, such materials may be included in the absorbent core, e.g., in form of super absorbing particles, or mixed with or built-into the core, e.g., cyclodextrines, and in addition various disinfectants. On the other hand, perfumed materials may be included in the absorbent article which lead to release of an agreeable odour, e.g., once the article has been wetted. Such materials may be included in e.g., the absorbent core.

The topsheet 11 is compliant, soft feeling, and non-irritating to the wearer's skin. Further, the topsheet 11 is liquid pervious permitting liquids to readily penetrate through its thickness. A suitable topsheet may be manufactured from a wide range of materials, such as porous foams, reticulated foams, apertured plastic films, natural fibres (e.g., wood or cotton fibres), synthetic fibres (e.g., polyester or polypropylene fibres) or from a combination of natural and synthetic fibres. Preferably, it is made of a hydrophobic material to isolate the wearer's skin from liquids in the absorbent core 13.

There are a number of manufacturing techniques which may be used to manufacture the topsheet 11. For example, the topsheet 11 may be woven, non-woven, spunbonded, air-through bonded, carded, or the like. A preferred topsheet 11 may be a bonded-carded web, such as carded and thermally bonded by means well known to those skilled in the fabrics art. Preferably, the topsheet 11 has a weight from 12 to 25 grams per square meter, a minimum dry tensile strength of at least 400 grams per centimetre in the machine direction and a wet tensile strength of at least 55 grams per centimetre in the cross machine direction.

One shall appreciate that the above describes one common arrangement of an absorbent undergarment, and that any conventional configuration of absorbent undergarments may be adopted to perform according to the invention when provided with the configuration of elastic members as taught herein. By means of example and not limitation, an absorbent article as intended herein may also include such undergarments which comprise an outer wrapper, typically a textile or textile-resembling outer wrapper, and attached on the inner side of said wrapper with respect to the wearer an absorbent unit. The absorbent unit may be generally comprised of a laminate of an absorbent core enclosed between a liquid pervious body-side liner or topsheet, and liquid impervious backsheet, essentially as disclosed herein. Advantageously, the top- and backsheets of said absorbent unit can be sized to allow for sealing the absorbent core there between, but to otherwise substantially correspond to the dimensions of the absorbent core. This allows to save material upon production and distribute the production. It shall also be understood that in the latter configuration, the core shaping elastics may be provided either on the outer wrapper or on the absorbent unit (e.g., on the body-side liner, on the backsheet or there between).

The elastic members 16, 17, 18, 19 and 20 can be made of natural or synthetic rubber and may also comprise any heat shrinkable elastic material as is well known in the art. Other suitable elastic members may comprise a wide variety of materials as are well known in the art including elastomeric films, polyurethane films, elastomeric foams, and formed elastic scrim. In addition, the elastic members may take a multitude of configurations. For example, the width of the elastic members 16, 17, 18, 19 and 20 may be varied from 0.25 mm to 25 mm or more; the elastic members may comprise a single strand of elastic material or may comprise several parallel or non-parallel strands of elastic material. When individual strands of elastic are used, the elastic members 16, 17, 18, 19 and 20 may include any suitable number of elastic strands. For example, the elastic members may include from about 1 to about 10 elastic strands. As a non-limiting example, suitable elastic strands include Filabell round section spandex yarn in 800 dtex, commercially available from the Albis Group.

The elastic members may be typically elongated prior to being attached to the respective portions of the absorbent article 1. For example, the elastic members may be elongated to at least about 1,2-fold and preferably to at least about 1,5-fold their relaxed length, such as from about 1,5 fold or about 2-fold to about 4-fold their relaxed length, before being attached such that the elastic members gather the material of the absorbent article 1 when relaxed, inter alia creating the crotch cup. The elastic members may be joined to the article 1 by any means known to those skilled in the art. For example, adhesive, thermal or ultrasonic bonding techniques or a combination thereof may be used to join the elastic members to the article 1. As a non-limiting example, a suitable adhesive includes Bostik H246504 hot melt adhesive which is commercially available from Bostik, Inc.

The barrier cuffs 21 provide a barrier to restrain the free flow of body exudates along the liquid-receiving surface 11 and provide a structure to hold and contain such exudates within the undergarment 1. The barrier cuffs 21 may be manufactured from a wide variety of materials such as polypropylene, polyester, rayon, nylon, foams, plastic films, formed films, and elastic foams. A number of manufacturing techniques may be used to manufacture the barrier cuffs. For example, the barrier cuffs may be woven, non-woven, spunbonded, carded, cast, blown or the like. In a preferred embodiment, the barrier cuffs contain fifth elastic members 22, to create an upstanding effect of the cuff so that exudates, especially loose faecal material which is not easily absorbed and tends to float along the liquid-receiving surface 11, will contact the cuffs 21, thus creating a restraint against the flow of body exudates.

Referring to the illustrative figures, the invention thus generally provides a disposable absorbent undergarment comprising: a liquid-permeable topsheet (11); a liquid-impermeable backsheet (12); a liquid-absorbent core (13) between the topsheet and the backsheet; a laminate comprising the topsheet, the backsheet and the core and having a front section (7), a rear section (8) and a crotch section (14) interposed between the front section and the rear section; first and second leg-hole defining edges (15) in the form of generally circular-arc-shaped notches on transversely opposite sides of the crotch section; stretchable elastic members (16, 17, 18) generally extending along each of the first and second leg-hole defining edges; the elastic members comprising first and second front-half elastic portions (16a, 16b) extending primarily along first and second front-halves (14a) of the first and second leg-hole defining edges, first and second rear-half elastic portions (17a, 17b) extending primarily along first and second rear-halves (14b) of the first and second leg-hole defining edges, and first and second core shaping elastic portions (18a, 18b) extending longitudinally on transversely opposite sides of the crotch section.

In an embodiment according to the invention, said first core shaping elastic portion (18a) does not intersect with any one of said first front-half and first rear-half elastic portions (16a, 17a), and said second core shaping elastic portion (18b) does not intersect with any one of said second front-half and second rear-half elastic portions (16b, 17b). In this embodiment, the presence of several non-elasticised areas achieves formation of the crotch cup and good aeration of the crotch region.

Referring to Fig. 3, the front-half elastic members 16 create first and second front-half elastic portions 16a and 16b. The elasticised sections 16a and 16b extend primarily along front-halves 14a of the leg-hole defining edges 15a, 15b. Similarly, the rear-half elastic members 17 create first and second rear-half elasticised sections 17a and 17b. The elasticised sections 17a and 17b extend primarily along rear-halves 14b of the leg-hole defining edges 15a, 15b. For good fit around the wearer's legs and improved leakage resistance against loose faecal material and gushes of urine, the leg-hole elastic members 16a, 16b, 17a and 17b creating leg-hole elasticised areas are attached in a semi-circular, curved or horseshoe-like pattern around the leg opening defining edges 15a and 15b. The core shaping elastic members 18 create first and second core shaping elasticised sections 18a and 18b. The core shaping elasticised sections extend primarily in the direction of the longitudinal centreline AA' of the undergarment.

Preferably, the core shaping elasticised sections 18a and 18b have a length of or extend over at least 5%, e.g., ≥ 6%, ≥ 7%, ≥ 8% or ≥ 9%, more preferably at least 10%, e.g., ≥ 11%, ≥ 12%, ≥ 13% or ≥ 14%, or at least 15%, e.g., ≥ 16%, ≥ 17%, ≥ 18%, ≥ 19% or ≥ 20%, and possibly at least ≥ 30%, ≥ 40%, ≥ 50% or more of the length of the crotch section 14 when extended. For example, the core shaping elasticised sections 18a and 18b may have a length of or extend over between about 1% and 75%, or between about 5% and 75%, or between about 1% and 50%, or between about 1% and 20%, such as between about 5% and 15%, or about 10% of the length of the crotch section 14 when extended. For example, the core shaping elasticised sections 18a and 18b may have a length in the longitudinal direction of between about 3 and about 15 cm, such as, e.g., between about 5 and about 12 cm, or between about 8 and 10 cm when extended.

In preferred embodiments, the core shaping elasticised sections 18a and 18b may have a length (*i.e.,* longitudinal length substantially along the A-A' axis) of or extend over at least about 50% or at least about 60%, more preferably at least about 70% or at least about 75%, even more preferably at least about 80% or at least about 85%, yet more preferably at least about 90% or at least about 95%, such as substantially over 100%, of the length of the crotch section 14 when extended. For example, in preferred embodiments the core shaping elasticised sections 18a and 18b may have a length (*i.e.,* longitudinal length substantially along the A-A' axis) of or extend over at least about 50% or at least about 60%, more preferably at least about 70% or at least about 75%, even more preferably at least about 80% or at least about 85%, yet more preferably at least about 90% or at least about 95%, such as substantially over 100%, of the length of the absorbent core or of the absorbent unit of the absorbent article as taught herein when extended.

For example, the core shaping elasticised sections 18a and 18b may have a length in the longitudinal direction of at least about 10 cm or at least about 15 cm, preferably at least about 20 cm or at least about 25 cm, more preferably at least about 30 cm or at least about 35 cm, even more preferably at least about 40 cm or at least about 45 cm, or even at least about 50 cm, when extended.

This allows the core shaping elasticised sections 18a and 18b to shape the crotch area to form a containment pocket and advantageously to also ensure good adherence of the longitudinally central portion of the absorbent article delimited by said elasticised sections 18a and 18b to the body of the user. This arrangement effectively guides the body discharge towards the crotch pocket while reducing any discharge leakage towards the leg openings.

Preferably, the core shaping elasticised sections 18a and 18b may be positioned on the garment longitudinally centrally, i.e., extending longitudinally to substantially similar extent on each side of the transverse midline BB' of the absorbent article. For instance, between no more than 70% and no less than 30%, or between no more than 60% and no less than 40% or about 50% of the length of the core shaping elasticised sections 18a and 18b may extend toward the front or rear portions of the garment with respect to the transverse centreline BB'.

Being enabled to contract, the core shaping elastic sections will cause the crotch area of the undergarment to take on a cup shape. A cup is formed due to the fact that the retraction forces of the elasticised sections 18a and 18b cause the product to curl around the transversal centreline BB'. Due to the interaction of the retraction force of elasticised sections 18a and 18b and the forces in the area in between elasticised sections 18a and 18b, the product also curls around longitudinal centreline AA'. This effect is even reinforced by the fifth elastic members in the barrier cuffs although these are not necessary for the purpose of this invention. The person skilled in the art will make the proper raw material and manufacturing parameters choice so as to optimise cup formation.

The first and second front-half elasticised sections 16a and 16b do not connect or intersect with first and second core shaping sections 18a and 18b, thus creating non-elasticised areas 23a and 23b, respectively. Similarly, the first and second rear-half elasticised sections 17a and 17b do not connect with first and second core shaping sections 18a and 18b, thus creating non-elasticised areas 24a and 24b, respectively. These non-elasticised areas 23 and 24 allow for an efficient cup formation and for air exchange between the inside of the undergarment and the environment.

As intended herein, the reference to intersection of elastic members, areas or sections refers to such contact or intersection as perceived when the absorbent garment is viewed as exemplified in Fig. 3, i.e., in plan view of the article in open configuration. Hence, intersecting elastic elements need not in fact be in direct physical contact or interaction with each other (e.g., they may be provided on different layers of the material forming the absorbent garment), but would be generally appreciated as overlapping or intersecting in plan view. Hence, as intended herein each core shaping elastic member 18 intersects with none (as illustrated in Fig. 3) of the front- and rear-half elastic members 16, 17. For example, a non-intersecting end of the core shaping elastic member 18 may be between about 0,1 cm and about 15 cm, or between about 0,2 cm and about 15 cm, or between about 0,5 cm and about 12 cm, or between about 1 cm and about 10 cm, or between about 5 cm and about 10 cm distant from the closest point of the closest front- or rear-half elastic member 16, 17, as measured in open configuration through the non-elasticised areas 23, 24.

In an embodiment, such as shown in Fig. 3, the longitudinally extending core-shaping elastic members 18 extend parallel to the longitudinal axis AA'. In a further embodiment, such as shown in Fig. 3, the longitudinally extending core-shaping elastic members 18 extend straight and parallel to the longitudinal axis AA'. However, it shall be appreciated that said core-shaping elastic members 18 may extend in a variety of shapes, forms and directions compatible with the denotation longitudinal extension. By means of example and not limitation, the core-shaping elastic members 18 may be generally skewed, sinusoidal, arc-shaped, circular, ellipsoid, etc., such as illustrated in preset Figures. Alternatively or in addition, the core-shaping elastic members 18 may be comprised of a plurality of intersecting (overlapping) or non-intersecting (non-overlapping) elastic elements, such as illustrated in preset Figures.

The core shaping elastic members 18a, 18b extend longitudinally on transversely opposite sides 15a, 15b of the crotch section. This may preferably denote that neither of said core shaping elastic members 18a, 18b overlaps with, intersects with or crosses the longitudinal centreline AA' of the absorbent article (such as, e.g., Fig. 3). More preferably, the core shaping elastic members 18a, 18b may be confined, in the transverse direction, to the outer (transversely distal) 70%, or 60%, even more preferably 50%, or 40%, or 30%, or 20% of the distance between the longitudinal centreline AA' and the respective crotch side edges 15a, 15b in the narrowest points of the latter.

In a further preferred embodiment, the core shaping elastic members 18a, 18b are located transversely outwardly from the absorbent core 13.

In embodiment as shown in Fig. 3, the first and second front-half elastic members 16a, 16b, and particularly the longitudinally central (i.e., proximal) ends thereof, are not connected through the crotch region (particularly not elastically connected, such as, for example, by means of a further transverse elastic member). Also, in an embodiment, the first and second rear-half elastic members 17a, 17b, and particularly the longitudinally central (i.e., proximal) ends thereof, are not connected through the crotch region (particularly not elastically connected, such as, for example, by means of a further transverse elastic member).

This arrangement may be achieved, for example, by applying and securing the first and second front-half elastic members 16a, 16b, and the first and second rear-half elastic members 17a, 17b to the absorbent article each as a separate element.

Alternatively, this arrangement may also be achieved by applying the front-half elastic members 16a, 16b, or the rear-half elastic members 17a, 17b, as single front or rear elastic elements, respectively, wherein in said front elastic element the longitudinally proximal ends of the elastic members 16a, 16b are connected by an interposing front elastic member running transversely through the crotch region, and/or wherein in the rear elastic element the longitudinally proximal ends of the elastic members 17a, 17b are connected by an interposing rear elastic member running transversely through the crotch region. Then, at least the portions of said front and rear elastic elements corresponding to the resulting front-half elastic members 16a, 16b and rear-half elastic members 17a, 17b are secured (such as, e.g., glued) to the absorbent article, and the respective interposing front and back elastic members are disconnected there from and disintegrated, such as for example by introducing a plurality of cuts therein. Thereby, the interposing portions are wholly (see, e.g., Fig. 3) or partly (see, e.g., Fig 11, T26 and T27) removed, such that their functionality as an elastic member is reduced, severed or eliminated. It shall be understood that remainders of said interposing portions may be kept in place and need not be removed from the article, since and insofar they do not exert an appreciable overall pull. The manner described in this paragraph may be more production cost effective, since it may be usually easier to initially apply the front-half elastic members 16a, 16b, and/or the rear-half elastic members 17a, 17b, as continuous elastic elements connected through the crotch region by front and rear elastic members, respectively.

The inventors have found that an arrangement wherein the first and second front-half elastic members 16a, 16b, and/or the first and second rear-half elastic members 17a, 17b, are not connected through the crotch region by said interposing members puts less constraints on the wearer and cooperates well with the crotch cup formed by the core shaping elastic members 18a, 18b. Such arrangement also allows for better aeration of the crotch region, and reduces or prevents transverse deformation of the crotch region.

Accordingly, an aspect of the invention also provides a disposable absorbent undergarment comprising: a liquid-permeable topsheet; a liquid-impermeable backsheet; a liquid-absorbent core between the topsheet and the backsheet; a laminate comprising the topsheet, the backsheet and the core and having a front section, a rear section and a crotch section interposed between the front section and the rear section; first and second leg-hole defining edges in the form of generally circular-arc-shaped notches on transversely opposite sides of the crotch section; stretchable elastic members generally extending along each of the first and second leg-hole defining edges; the elastic members comprising first and second front-half elastic portions extending primarily along first and second front-halves of the first and second leg-hole defining edges, first and second rear-half elastic portions extending primarily along first and second rear-halves of the first and second leg-hole defining edges, and first and second core shaping elastic portions extending longitudinally on transversely opposite sides of the crotch section; wherein either the first and second front-half elastic portions, or the first and second rear-half elastic portions, or neither, are connected through the crotch region by an interposing elastic member. In said aspect, the absence of said interposing elastic member(s) between one or both of the front- and rear-half elastic members 16a, 16b and 17a, 17b achieves better aeration of the product, whereby it need not be essential to avoid intersection between said core shaping elastic portions 18a, 18b and said front- and/or rear-half elastic portions 16a, 16b and 17a, 17b (see embodiment in Fig. 9).

In another embodiment, such as illustrated in Fig. 10, the first and second front-half elastic members 16a, 16b, and particularly the longitudinally central (i.e., proximal) ends thereof, may be connected through the crotch region (particularly elastically connected, such as, for example, by means of a further transverse elastic member 16c). Also, in an embodiment, the first and second rear-half elastic members 17a, 17b, and particularly the longitudinally central (i.e., proximal) ends thereof, may be connected through the crotch region (particularly elastically connected, such as, for example, by means of a further transverse elastic member 17c).

With reference to Fig. 11, embodiments T17 to T20, the invention also contemplates asymmetric arrangements, wherein only the first and second front-half elastic members 16a, 16b are connected through the crotch region, particularly by means of a further transverse elastic member 16c, whereas the first and second rear-half elastic members 17a, 17b are not so connected; or asymmetric arrangements wherein only the first and second rear-half elastic members 17a, 17b are connected through the crotch region, particularly by means of a further transverse elastic member 17c, whereas the first and second front-half elastic members 16a, 16b are not so connected. Such arrangements achieve good aeration, while they also accommodate better fit with the legs and the asymmetric movements thereof.

In an alternative embodiment, the core shaping elastic portions 18a, 18b are positioned transversely inwardly (i.e., on the inside or more proximal relative to the longitudinal centreline AA') from the front- and rear-half elastic members 16 and 17. In another alternative embodiment, the core shaping elastic portions 18a, 18b are also positioned longitudinally inwardly (i.e., on the inside or more proximal relative to the transverse centreline AA') from the front- and rear-half elastic members 16 and 17. Each of said arrangements or a combination thereof (see Fig. 3) avoids intersection of the respective elastic portion(s).

Preferably, as shown in Figures 3 a-f the intersection between the core shaping elastic portions 18 and the respective front- and/or rear-half elastic members 16 and 17 may be avoided by positioning the core shaping elastic portions 18 transversely inwardly *(i.e.,* on the inside or more proximal relative to the longitudinal centreline AA') from the front- and rear-half elastic members 16 and 17. This arrangement allows to include comparably longer core shaping elastic portions 18a and/or 18b as for example illustrated in Figures 3a-f, particularly where the front- and/or rear-half elastic members 16 and 17 are not connected through the crotch region by an interposing elastic member, whereby said intersection may be avoided. Hence, the elastic portions 18a and/or 18b need not be positioned longitudinally inwardly from the respective front- and/or rear-half elastic members 16, 17.

In embodiments according to the invention, the core-shaping elastic portions 18a and/or 18b may extend longitudinally beyond the crotch-ends of the front- and/or rear-half elastic portions 16, 17. For example, with reference to Figures 3a-f, the core-shaping elastic portions 18a and/or 18b may extend longitudinally beyond or outwardly relative to the crotch ends of the front- and/or rear-half elastic portions 16, 17, for example may so extend over at least about 10% or at least about 20%, preferably over at least about 30% or at least about 40%, more preferably over at least about 50% or at least about 60%, yet more preferably over at least about 70% or at least about 80%, and also more preferably over at least about 90% or over up to 100% of the longitudinal distance (*i.e.,* distance along the axis A-A') as defined by the front- and/or rear-half elastic portions 16, 17. In this context, the longitudinal distance defined by each front-half elastic portion 16 refers to the distance or segment along the A-A' axis from the front end to the crotch end of said front-half elastic portion 16, and the longitudinal distance defined by each rear-half elastic portion 17 refers to the distance or segment along the A-A' axis from the crotch end to the rear end of said rear-half elastic portion 17. In embodiments the core-shaping elastic portions 18a and/or 18b may even extend longitudinally beyond or outwardly relative to the front end of the front-half elastic portions 16 and/or the rear end of the rear-half elastic portions 17.

As explained such comparably longer core shaping elasticised sections 18a and 18b allow to shape the crotch area to form a containment pocket (particularly as they do not intersect with the leg-opening elastics) and advantageously also ensure good adherence of the longitudinally central portion of the absorbent article delimited by said elasticised sections 18a and 18b to the body of the user. This arrangement effectively guides the body discharge towards the crotch pocket while reducing any discharge leakage towards the leg openings.

Accordingly, in an aspect the invention provides a disposable absorbent undergarment comprising: a liquid-permeable topsheet 11; a liquid-impermeable backsheet 12; a liquid-absorbent core 13 between the topsheet and the backsheet; a laminate comprising the topsheet, the backsheet and the core and having a front section 7, a rear section 8 and a crotch section 14 interposed between the front section and the rear section; first and second leg-hole defining edges 15 in the form of generally circular-arc-shaped notches on transversely opposite sides of the crotch section; stretchable elastic members 16, 17, 18 generally extending along each of the first and second leg-hole defining edges; the elastic members comprising first and second front-half elastic portions 16a, 16b extending primarily along first and second front-halves 14a of the first and second leg-hole defining edges, first and second rear-half elastic portions 17a, 17b extending primarily along first and second rear-halves 14b of the first and second leg-hole defining edges, and first and second core shaping elastic portions 18a, 18b extending longitudinally on transversely opposite sides of the crotch section, wherein said first core shaping elastic portion 18a does not intersect with any one of said first front-half and first rear-half elastic portions 16a, 17a, and wherein said second core shaping elastic portion 18b does not intersect with any one of said second front-half and second rear-half elastic portions 16b, 17b, characterised in that said first core-shaping elastic portion 18a extends longitudinally beyond the crotch ends of the first front-half and first rear-half elastic portions 16a, 17a, and the second core-shaping elastic portion 18b extends longitudinally beyond the crotch ends of the second front-half and second rear-half elastic portions 16b, 17b.

In a further embodiment, as also shown in Fig. 3, the elastic members 22 of the upstanding cuffs 21 do not intersect with said first and second front-half and first and second rear-half elastic portions 16, 17. In a further embodiment, as also shown in Fig. 3, the upstanding cuffs 21 do not intersect with said first and second front-half and first and second rear-half elastic portions 16, 17. This preserves non-elasticised areas which achieve the desired effects of the invention. In an example, the elastic members 22 of the upstanding cuffs 21 or the cuffs per se are positioned transversely inwardly (i.e., on the inside or more proximal relative to the longitudinal centreline AA') from the front- and rear-half elastic members 16 and 17.

Referring to Fig. 4, further elastic members creating additional elasticised sections 41 and 42 have been added to the core shaping section so as to optimise cup formation. Elasticised sections 18a and 18b, extending primarily in the direction of the longitudinal centreline AA' work in combination with elasticised sections 41 and 42, extending primarily in the direction of transversal centreline BB' so as to form a cup by creating areas of higher elevation around a depression in the crotch area of the disposable absorbent undergarment.

Referring to Fig 5, the elastic members of the core shaping section are positioned so that elasticised sections 18a and 18b contact to form one single core shaping elasticised section 18.

Figure 11 illustrates various arrangements of the core shaping portions 18 and the front- and rear elastic portions 16, 17 according to the invention (exemplary embodiments T1 to T27).

A further advantageous arrangement of the crotch and/or leg elastic members is illustrated in an expanded view in Figure 12 in connection with the embodiment T25, wherein irregularly or unequally spaced gaps in the elastic members or strands thereof further hinder the escape of liquids (dashed arrow) from the crotch region through the edges 15.

Referring to fig 8, an alternative embodiment with improved airflow and cup formation support for the non-elasticised sections 23, 24 is shown. In this case the backsheet 4 is composed as a laminate of an upper backsheet 51 and a lower backsheet 52. Both are attached to each other by patches of pressure-sensitive adhesive 53, although other chemical, mechanical or thermal means of sealing two materials can be used. The upper backsheet 51 is oversized vs. lower backsheet 52, thus creating channels 54. The channels 54 allow for an improved airflow and provide a buffering effect for the tensions arising from either the leg opening elasticised sections and/or the core shaping elasticised sections, thus allowing the latter to act freely and provide optimal cup formation.

The invention also broadly covers methods of manufacture and apparatuses specifically configured to manufacture the herein disclosed absorbent articles. Generally, said methods may comprise steps of providing and combining, connecting, attaching or fastening in the herein explained configurations the various elements comprised in absorbent articles taught herein. Apparatuses may comprise means for supplying or feeding and combining, connecting, attaching or fastening in the herein explained configurations the various elements comprised in absorbent articles taught herein.

## Claims

1. A disposable absorbent undergarment comprising: a liquid-permeable topsheet (11); a liquid-impermeable backsheet (12); a liquid-absorbent core (13) between the topsheet and the backsheet; a laminate comprising the topsheet, the backsheet and the core and having a front section (7), a rear section (8) and a crotch section (14) interposed between the front section and the rear section; first and second leg-hole defining edges (15) in the form of generally circular-arc-shaped notches on transversely opposite sides of the crotch section; stretchable elastic members (16, 17, 18) generally extending along each of the first and second leg-hole defining edges; the elastic members comprising first and second front-half elastic portions (16a, 16b) extending primarily along first and second front-halves (14a) of the first and second leg-hole defining edges, first and second rear-half elastic portions (17a, 17b) extending primarily along first and second rear-halves (14b) of the first and second leg-hole defining edges, and first and second core shaping elastic portions (18a, 18b) extending longitudinally on transversely opposite sides of the crotch section, wherein said first core shaping elastic portion (18a) does not intersect with any one of said first front-half and first rear-half elastic portions (16a, 17a), and wherein said second core shaping elastic portion (18b) does not intersect with any one of said second front-half and second rear-half elastic portions (16b, 17b), **characterised in that** said first core-shaping elastic portion (18a) extends longitudinally beyond the crotch end of the first front-half and/or first rear-half elastic portions (16a, 17a), and the second core-shaping elastic portion (18b) extends longitudinally beyond the crotch end of the second front-half and/or second rear-half elastic portions (16b, 17b).

2. The disposable absorbent undergarment according to claim 1, wherein said first core-shaping elastic portion (18a) extends longitudinally beyond the crotch ends of the first front-half and first rear-half elastic portions (16a, 17a), and the second core-shaping elastic portion (18b) extends longitudinally beyond the crotch ends of the second front-half and second rear-half elastic portions (16b, 17b).

3. The disposable absorbent undergarment according to any one of claims 1 or 2, wherein the core shaping elastic portions (18a, 18b) are positioned transversely inwardly from the front- and rear-half elastic members (16, 17).

4. The disposable absorbent undergarment according to any one of claims 1 to 3, wherein the first and second front-half elastic portions (16a, 16b) and the first and second rear-half elastic portions (17a, 17b) are not connected through the crotch region by an interposing elastic member (16c, 17c).

5. The disposable absorbent undergarment according to any one of claims 1 to 3, wherein the core-shaping elastic portions (18a, 18b) extend longitudinally beyond the crotch ends of the front- and/or rear-half elastic portions (16, 17) and over at least about 50% more preferably over at least about 75% of the longitudinal distance defined by said front- and rear-half elastic portions (16, 17).

6. The disposable absorbent undergarment according to any one of claims 1 to 5, wherein the core shaping elastic portions (18a, 18b) extend parallel to the longitudinal axis (AA').

7. The disposable absorbent undergarment according to any of claims 1 to 6, wherein the core shaping elastic portions (18a, 18b) extend over at least about 50% more preferably at least about 75% of the length of the crotch section (14) when extended.

8. The disposable absorbent undergarment according to any of claims 1 to 7, wherein the core shaping elastic portions (18a, 18b) have a length in the longitudinal direction of at least about 30 cm preferably at least about 40 cm when extended.

9. The disposable absorbent undergarment according to any of claims 1 to 8, wherein the core shaping elastic portions (18a, 18b) are positioned longitudinally centrally on the undergarment.

10. The disposable absorbent undergarment according to any of claims 1 to 9, wherein neither of said core shaping elastic members (18a, 18b) intersects with the longitudinal centreline (AA').

11. The disposable absorbent undergarment according to any of claims 1 to 10, wherein the core shaping elastic members (18a, 18b) are confined, in the transverse direction, to the outer 50% of the distance between the longitudinal centreline AA' and the respective crotch side edges (15a, 15b) in the narrowest points of the latter.

12. The disposable absorbent undergarment according to any of claims 1 to 11, wherein the core shaping elastic members (18a, 18b) are located transversely outwardly from the absorbent core (13).

13. The disposable absorbent undergarment according to any of claims 1 to 12, further comprising upstanding cuffs (21) comprising elastic members (22), and wherein the upstanding cuffs (21) or at least the elastic members (22) of said upstanding cuffs (21) do not intersect with said first and second front-half and first and second rear-half elastic portions (16, 17).

14. A method for manufacture of the disposable absorbent undergarment according to any of claims 1 to 13 comprising providing and combining, connecting, attaching or fastening the elements comprised in absorbent articles as defined in any one of claims 1 to 13 in the configuration as defined in claims 1 to 13.

## Patentansprüche

1. Eine absorbierende Einweg-Unterwäsche umfassend: eine flüssigkeitsdurchlässige Oberschicht (11); eine flüssigkeitsundurchlässige Rückenschicht (12); einen flüssigkeitsabsorbierenden Kern (13) zwischen der Oberschicht und der Rückenschicht; ein Laminat, das die Oberschicht, die Rückenschicht und den Kern umfasst, und mit einem vorderen Abschnitt (7), einem hinteren Abschnitt (8) und einem Schrittabschnitt (14), der zwischen dem vorderen Abschnitt und dem hinteren Abschnitt positioniert ist; erste und zweite Beinöffnung definierende Kanten (15) in der Form von im allgemeinen kreisbogenförmigen Kerben, auf quer gegenüberliegenden Seiten des Schrittabschnitts; ausdehnbare elastische Elemente (16, 17, 18), die sich im allgemeinen entlang jeder der ersten und zweiten Beinöffnung definierenden Kanten erstrecken; die elastischen Elemente umfassend erste und zweite elastische Teile (16a, 16b) der vorderen Hälfte, die sich hauptsächlich entlang ersten und zweiten vorderen Hälften (14a) der ersten und zweiten, die Beinöffnung definierenden Kanten erstrecken, erste und zweite elastische Teile (17a, 17b) der hinteren Hälfte, die sich hauptsächlich entlang ersten und zweiten hinteren Hälften (14b) der ersten und zweiten, die Beinöffnung definierenden Kanten erstrecken, und erste und zweite kernformende elastische Teile (18a, 18b), die sich längs auf quer gegenüberliegenden Seiten des Schrittabschnitts erstrecken, wobei das erste kernformende elastische Teil (18a) keine der elastischen Teile (16a, 17a) der ersten vorderen Hälfte und ersten hinteren Hälfte schneidet, und wobei das zweite kernformende elastische Teil (18b) keine der elastischen Teile (16b, 17b) der zweiten vorderen Hälfte und zweiten hinteren Hälfte schneidet, **dadurch gekennzeichnet, dass** das erste kernformende elastische Teil (18a) sich längs über das Schrittende der elastischen Teile (16a, 17a) der ersten vorderen Hälfte und/oder der ersten hinteren Hälfte erstreckt, und das zweite kernformende elastische Teil (18b) sich längs über das Schrittende der elastischen Teile (16b, 17b) der zweiten vorderen Hälfte und/oder zweiten hinteren Hälfte erstreckt.

2. Die absorbierende Einweg-Unterwäsche nach Anspruch 1, wobei das erste kernformende elastische Teil (18a) sich längs über die Schrittenden der elastischen Teile (16a, 17a) der ersten vorderen Hälfte und ersten hinteren Hälfte erstreckt, und das zweite kernformende elastische Teil (18b) sich längs über die Schrittenden der elastischen Teile (16b, 17b) der zweiten vorderen Hälfte und zweiten hinteren Hälfte erstreckt.

3. Die absorbierende Einweg-Unterwäsche nach einem der Ansprüche 1 oder 2, wobei die kernformende elastische Teile (18a, 18b) quer nach innen von den elastischen Elementen (16, 17) der vorderen und hinteren Hälfte positioniert sind.

4. Die absorbierende Einweg-Unterwäsche nach einem der Ansprüche 1 bis 3, wobei die erste und zweite elastische Teile (16a, 16b) der vorderen Hälfte und die erste und zweite elastische Teile (17a, 17b) der hinteren Hälfte nicht miteinander verbunden sind durch den Schrittbereich mittels eines zwischengeschalteten elastischen Elements (16c, 17c).

5. Die absorbierende Einweg-Unterwäsche nach einem der Ansprüche 1 bis 3, wobei die kernformende elastische Teile (18a, 18b) sich längs über die Schrittenden der elastischen Teile (16, 17) der vorderen und hinteren Hälfte erstrecken und über mindestens etwa 50%, mehr bevorzugt über mindestens etwa 75% des Längsabstands, der durch die elastische Teile (16, 17) der vorderen und hinteren Hälfte definiert wird.

6. Die absorbierende Einweg-Unterwäsche nach einem der Ansprüche 1 bis 5, wobei die kernformende elastische Teile (18a, 18b) sich parallel zur Längsachse (AA') erstrecken.

7. Die absorbierende Einweg-Unterwäsche nach einem der Ansprüche 1 bis 6, wobei die kernformende elastische Teile (18a, 18b) sich über mindestens etwa 50%, mehr bevorzugt mindestens etwa 75% der Länge des Schrittabschnitts (14) erstrecken, wenn erstreckt.

8. Die absorbierende Einweg-Unterwäsche nach einem der Ansprüche 1 bis 7, wobei die kernformende elastische Teile (18a, 18b) eine Länge in Längsrichtung von mindestens etwa 30 cm, vorzugsweise mindestens etwa 40 cm aufweisen, wenn erstreckt.

9. Die absorbierende Einweg-Unterwäsche nach einem der Ansprüche 1 bis 8, wobei die kernformende elastische Teile (18a, 18b) in Längsrichtung zentral auf der Unterwäsche positioniert sind.

10. Die absorbierende Einweg-Unterwäsche nach einem der Ansprüche 1 bis 9, wobei keine der kernformenden elastischen Elemente (18a, 18b) die Längsmittellinie (AA') schneidet.

11. Die absorbierende Einweg-Unterwäsche nach einem der Ansprüche 1 bis 10, wobei die kernformende elastische Elemente (18a, 18b), in der Querrichtung, begrenzt sind, zu dem äußeren 50% des Abstandes zwischen der Längsmittellinie AA' und den jeweiligen Schrittseitenkanten (15a, 15b) in den engsten Stellen der letzteren.

12. Die absorbierende Einweg-Unterwäsche nach einem der Ansprüche 1 bis 11, wobei die kernformende elastische Elemente (18a, 18b) sich quer nach außen von dem absorbierenden Kern (13) befinden.

13. Die absorbierende Einweg-Unterwäsche nach einem der Ansprüche 1 bis 12, die ferner aufrecht stehende Bündchen (21) umfassend elastische Elemente (22) umfasst, und wobei die aufrecht stehende Bündchen (21) oder mindestens die elastische Elemente (22) der aufrecht stehenden Bündchen (21) die elastische Teile der ersten und zweiten vorderen Hälfte und die elastische Teile der ersten und zweiten hinteren Hälfte (16, 17) nicht schneiden.

14. Ein Verfahren zur Herstellung der absorbierenden Einweg-Unterwäsche nach einem der Ansprüche 1 bis 13, umfassend das Bereitstellen und Kombinieren, Verbinden, Anbringen oder Befestigen der in absorbierenden Artikeln umfassten Elemente wie in einem der Ansprüche 1 bis 13 definiert in der Konfiguration wie in den Ansprüchen 1 bis 13 definiert.

## Revendications

1. Un sous-vêtement absorbant jetable comprenant: une feuille supérieure perméable aux liquides (11); une feuille inférieure imperméable aux liquides (12); un noyau absorbant les liquides (13) entre la feuille supérieure et la feuille inférieure; un stratifié comprenant la feuille supérieure, la feuille inférieure et le noyau et ayant une section avant (7), une section arrière (8) et une section d'entrejambe (14) interposée entre la section avant et la section arrière; des premiers et secondes bords (15) définissant des ouvertures pour les jambes sous la forme d'encoches généralement en forme d'arc de cercle sur des côtés transversalement opposés de la section d'entrejambe; des éléments élastiques extensibles (16, 17, 18) étendant généralement le long de chacun des premiers et secondes bords définissant des ouvertures pour les jambes; les éléments élastiques comprenant des premières et secondes parties élastiques (16a, 16b) de la moitié avant s'étendant principalement le long des premières et secondes moitiés avant (14a) des premiers et secondes bords définissant des ouvertures pour les jambes, des premières et secondes parties élastiques (17a, 17b) de la moitié arrière s'étendant principalement le long des premières et secondes moitiés arrière (14b) des premiers et secondes bords définissant des ouvertures pour les jambes, et des premières et secondes parties élastiques formant le noyau (18a, 18b) s'étendant longitudinalement sur des côtés transversalement opposés de la section d'entrejambe, dans lequel ladite première partie élastique formant le noyau (18a) ne croise pas avec l'une quelconque desdites parties élastiques (16a, 17a) de la première moitié avant et la première moitié arrière, et dans lequel ladite seconde partie élastique formant le noyau (18b) ne croise pas avec l'une quelconque desdites parties élastiques (16b, 17b) de la seconde moitié avant et la seconde moitié arrière, **caractérisé en ce que** ladite première partie élastique formant le noyau (18a) s'étend longitudinalement au-delà de l'extrémité d'entrejambe des parties élastiques de la première moitié avant et/ou la première moitié arrière (16a, 17a), et la seconde partie élastique formant le noyau (18b) s'étend longitudinalement au-delà de l'extrémité d'entrejambe des parties élastiques de la seconde moitié avant et/ou la seconde moitié arrière (16b, 17b).

2. Le sous-vêtement absorbant jetable selon la revendication 1, dans lequel ladite première partie élastique formant le noyau (18a) s'étend longitudinalement au-delà des extrémités d'entrejambe des parties élastiques de la première moitié avant et la première moitié arrière (16a, 17a), et la seconde partie élastique formant le noyau (18b) s'étend longitudinalement au-delà des extrémités d'entrejambe des parties élastiques de la seconde moitié avant et la seconde moitié arrière (16b, 17b).

3. Le sous-vêtement absorbant jetable selon l'une quelconque des revendications 1 ou 2, dans lequel les parties élastiques formant le noyau (18a, 18b) sont positionnées transversalement vers l'intérieur à partir des éléments élastiques de moitié avant et arrière (16, 17).

4. Le sous-vêtement absorbant jetable selon l'une quelconque des revendications 1 à 3, dans lequel les parties élastiques de la première et seconde moitié avant (16a, 16b) et les parties élastiques de la première et seconde moitié arrière (17a, 17b) ne sont pas reliées via la région d'entre-jambes par un élément élastique d'interposition (16c, 17c).

5. Le sous-vêtement absorbant jetable selon l'une quelconque des revendications 1 à 3, dans lequel les parties élastiques formant le noyau (18a, 18b) s'étendent longitudinalement au-delà des extrémités d'entrejambe des parties élastiques de la moitié avant et/ou arrière (16, 17) et sur au moins environ 50%, plus de préférence sur au moins environ 75% de la distance longitudinale définie par lesdites parties élastiques de la moitié avant et arrière (16, 17).

6. Le sous-vêtement absorbant jetable selon l'une quelconque des revendications 1 à 5, dans lequel les parties élastiques formant le noyau (18a, 18b) s'étendent parallèlement à l'axe longitudinal (AA').

7. Le sous-vêtement absorbant jetable selon l'une quelconque des revendications 1 à 6, dans lequel les parties élastiques formant le noyau (18a, 18b) s'étendent sur au moins environ 50%, plus de préférence au moins environ 75% de la longueur de la section d'entrejambe (14) lorsqu' étendu.

8. Le sous-vêtement absorbant jetable selon l'une quelconque des revendications 1 à 7, dans lequel les parties élastiques formant le noyau (18a, 18b) ont une longueur dans la direction longitudinale d'au moins environ 30 cm, de préférence d'au moins environ 40 cm, lorsqu' étendu.

9. Le sous-vêtement absorbant jetable selon l'une quelconque des revendications 1 à 8, dans lequel les parties élastiques formant le noyau (18a, 18b) sont positionnées longitudinalement centralement sur le sous-vêtement.

10. Le sous-vêtement absorbant jetable selon l'une quelconque des revendications 1 à 9, dans lequel aucuns desdits éléments élastiques formant le noyau (18a, 18b) croisent avec la ligne médiane longitudinale (AA').

11. Le sous-vêtement absorbant jetable selon l'une quelconque des revendications 1 à 10, dans lequel les éléments élastiques formant le noyau (18a, 18b) sont confinés, dans la direction transversale, à la partie 50% extérieur de la distance entre la ligne médiane longitudinale AA' et les bords latéraux d'entre-jambes respectifs (15a, 15b) dans les points les plus étroits de ceux-ci.

12. Le sous-vêtement absorbant jetable selon l'une quelconque des revendications 1 à 11, dans lequel les éléments élastiques formant le noyau (18a, 18b) sont situés transversalement vers l'extérieur à partir du noyau absorbant (13).

13. Le sous-vêtement absorbant jetable selon l'une quelconque des revendications 1 à 12, comprenant en outre des poignets dressés (21) comprenant des éléments élastiques (22), et dans lequel les poignets dressés (21) ou au moins les éléments élastiques (22) desdits poignets dressés (21) ne croisent pas avec lesdites parties élastiques de la première et seconde moitié avant et la première et seconde moitié arrière (16, 17).

14. Un procédé de fabrication du sous-vêtement absorbant jetable selon l'une quelconque des revendications 1 à 13, comprenant la fourniture et la combinaison, la connexion, la fixation ou le rattachement des éléments compris dans des articles absorbants tels que définis dans l'une quelconque des revendications 1 à 13 dans la configuration telle que définie dans les revendications 1 à 13.
